# EUROPÄISCHE PATENTSCHRIFT

(11) **EP 1 138 320 B1**
(45) Veröffentlichungstag und Bekanntmachung des Hinweises auf die Patenterteilung: **03.05.2006**
(21) Anmeldenummer: 01105545.6
(22) Anmeldetag: 06.03.2001
(51) Int. Cl.: A61K 9/22

(54) **Wirkstoffhaltige Schwimmformen enthaltend Polyvinylacetat und Polyvinylpyrrolidon, deren Verwendung und Herstellung**
Floating forms containing drug, polyvinyl acetate and polyvinyl pyrrolidone, their use and preparation
Formes flottantes contenant un pricipe actif, de l' acétate de polyvinyle et du polyvinylpyrrolidone, leur utilisation et leur préparation

(30) Priorität: 27.03.2000 DE 10014588
(43) Veröffentlichungstag der Anmeldung: 04.10.2001
(73) Patentinhaber: BASF Aktiengesellschaft, 67056 Ludwigshafen (DE)
(72) Erfinder: Kolter, Karl, Dr., 67117 Limburgerhof (DE); Schönherr, Michael, Dr., 67227 Frankenthal (DE); Ascherl, Hermann, 67246 Dirmstein (DE)

(56) Entgegenhaltungen:
- EP-A- 0 575 930
- EP-A- 0 669 129
- EP-A- 0 717 988

## Beschreibung

Die vorliegende Erfindung betrifft wirkstoffhaltige Darreichungsformen enthaltend Polyvinylacetat und Polyvinylpyrrolidon, die nach Einnahme auf dem Magensaft schwimmen und so zu einer verzögerten Wirkstofffreisetzung führen. Diese Formen können durch einfache Verfahren hergestellt werden und weisen außergewöhnliche mechanische Festigkeiten auf.

Retardierte Darreichungsformen gewinnen eine immer größere Bedeutung, zum einen, weil die Verabreichungshäufigkeit reduziert werden kann und zum anderen, weil sie zu einer Reduzierung der Blutspiegelschwankungen führen. Der kleinere maximale Blutspiegel kann die Schwere von dosisabhängigen Nebenwirkungen verringern und so beispielsweise bei Arzneimitteln die Verträglichkeit verbessern. Die größere minimale Plasmakonzentration erhöht die Wirksamkeit besonders von Wirkstoffen, bei denen eine bestimmte Konzentrationsschwelle nicht unterschritten werden sollte.

Nach der Einnahme der retardierten Darreichungsform gelangt sie in den Magen, wo sie normalerweise nach 0,5 - 3 h in den Dünndarm befördert wird. Die Dünndarmpassagezeit beträgt üblicherweise 3 - 6 h. Dies hat zur Folge, daß die Resorption des Wirkstoffes innerhalb von ca. 3 - 6 h abgeschlossen sein muß, weil die meisten Wirkstoffe im Colon nicht mehr oder nur in einem vernachlässigbarem Ausmaß resorbiert werden. Ein längerer Retardierungszeitraum ist daher nur schwer einstellbar. Bei Wirkstoffen, die in diesem Zeitraum nicht vollständig resorbiert werden, sinkt die Bioverfügbarkeit ab, weil ein Teil der Dosis verloren geht. Hinzu kommt, daß bestimmte Wirkstoffe im Dünndarm ein Resorptionsfenster besitzen, das bei herkömmlichen Darreichungsformen sehr schnell passiert wird.

Ein System, das längere Zeit im Magen verbleibt und kontinuierlich Wirkstoff abgibt, würde diese Nachteile umgehen, denn der Wirkstoff würde ständig in gelöster Form den Pylorus passieren und könnte im Dünndarm aufgenommen werden. So können einerseits die Bioverfügbarkeit, andererseits aber auch die Wirkdauer beispielsweise eines Arzneimittels verlängert werden.

Darüber hinaus gibt es einige Wirkstoffe, die lokal im Magen wirken sollen. Auch bei diesen wird häufig eine verlängerte Wirkdauer angestrebt.

Es gab bisher zahlreiche Ansätze, die Verweildauer durch Tabletten zu verlängern, die im Magen aufquellen und so groß werden, daß sie nicht mehr den Pylorus passieren können. Solche Formen werden beschrieben in US-Patent 4,871,548; 4,767,627; 5,443,843; 5,007,790; 4,851,232; WO 99/07342. Meistens werden hierbei hydroxyalkylierte Cellulosen als Quellmittel verwendet. Alle diese Formen haben den Nachteil, daß sie den Magenausgang verstopfen können und gesundheitliche Probleme verursachen können. Außerdem hängt die Quellung stark vom Mageninhalt und der Osmolarität des Mediums ab. Dadurch wird letzten Endes auch die Retardwirkung und die Verweilzeit beeinflußt.

Eine andere Möglichkeit die Verweilzeit im Magen zu verlängern, ist die Herstellung von Schwimmformen. Diese schwimmen auf dem Mageninhalt und werden, da sich der Pylorus im unteren Teil des Magens befindet, über längere Zeit nicht in den Dünndarm entleert.

Zur Herstellung von solchen Formen sind verschiedene Verfahren bekannt. So können Stoffe eingearbeitet werden, die per se eine niedrige Dichte aufweisen, wie z. B. Fette, Öle oder Wachse. Solche Formen sind in den Anmeldungen EP 198769 (Forest Laboratories Inc.), US 4,424,235, US Anm.nr. 834,347 (US 4,126,672), BE 839604 (Hoffmann-LaRoche) beschrieben. Dazu sind aber höhere Mengen erforderlich, die die Darreichungsformen volumenmäßig vergrößern und schwerer schluckbar machen, und außerdem haben diese Stoffe einen sehr nachteiligen Einfluß auf die Festigkeit der Formlinge. Bei der Verpressung entstehen Tabletten mit niedrigen Bruchfestigkeiten und die Tabletten kleben bei der Herstellung häufig am Stempel. In der Anmeldung DE 3527852 (Nippon Shinyaku KK) werden fetthaltigen Mischungen beschrieben, die in eine Kapsel gefüllt und zur Verfestigung erhitzt werden müssen. Dies ist langwierig und für temperaturlabile Wirkstoffe überhaupt nicht geeignet. Durch Kühlung und Gelierung und Trocknung werden Formlinge in der Anmeldung US 4,814,179 (Univ. of St.Johns) hergestellt. Dieses Verfahren ist noch deutlich aufwendiger.

Eine andere Methodik macht sich die Gasentwicklung aus Salzen der Kohlensäure zu nutzen. Hierbei werden diese Salze zusammen mit Gelbildnern in die Darreichungsformen eingearbeitet und nach Einwirkung der Magensäure entsteht CO₂, das die Form aufbläht und zum Aufschwimmen führt. Um unabhängig von der Magensäure zu sein, werden häufig physiologisch verträgliche Säuren, wie z. B. Zitronensäure oder Weinsäure eingearbeitet. Diese Zubereitungen sind sehr empfindlich gegenüber Feuchtigkeit, so daß die Herstellung bei niedriger Luftfeuchte erfolgen muß und keine wasserhaltigen Hilfsstoffe eingesetzt werden können. Das Packmaterial der Darreichungsformen muß sehr dicht sein, damit die Formen nicht schon während der Lagerung aufbrausen. Durch die Gasentwicklung bei Kontakt mit Säure wird oft das Gefüge der Darreichungsformen in Mitleidenschaft gezogen und der Retardeffekt verringert. Da diese Zubereitungen oft schlecht verpreßbar sind und keine Tabletten mit ausreichender mechanischer Stabilität erhalten werden, werden solche Zubereitungen häufig umständlich in Hart- oder Weichgelatinekapseln abgefüllt. Beispiele hierfür sind in den Anmeldungen GB 2283172 (Scherer LTD), GB 2283171 (Reckitt & Colman Prod LTD) beschrieben.

Die Herstellung von Tabletten ist beschrieben in WO 99/45887 (Univ Temple), US 4,167,558 (Hoffmann LaRoche), die Herstellung von Zweischichttabletten in US 4140755 (Hoffmann LaRoche). Neben den oben bereits erwähnten Nachteilen bereitet bei diesen Tabletten die Reproduzierbarkeit der Freisetzung enorme Probleme. Es ist allgemein bekannt, daß das Gelbildungsvermögen und die Gelstärke bei Polysacchariden aufgrund der Variation der Kettenlänge und des Substitutionsgrades von Charge zu Charge schwankt und dies wird noch verstärkt durch die Störung des Gelgerüstes durch die CO₂-Entwicklung. Die Gelbildner reagieren ferner auf unterschiedliche Osmolaritäten der Freisetzungsmedien sehr empfindlich mit Veränderungen in der Freisetzung.

Noch anfälliger und komplizierter sind Zubereitungen, bei denen Coatingschichten auf einen Kern, der CO₂ entwickeln kann, aufgebracht werden. Zum Teil enthält das Coating selbst ein Salz der Kohlensäure. Diese Coatings müssen unter Verwendung organischer Lösungsmittel aufgebracht werden. Solche Zubereitungen sind beschrieben in EP 235718 (Eisai KK), US 4,101,650 Microbiochemical Research Foundation), WO 99/49868 (Yuhan Corp).

Ein in eine Kapsel abgefülltes Pulver mit Wirkstoff, Hydrokolloid, pH-abhängigem Polymer und Bindemittel wird in US 5169638 (Squibb & Sons Inc) beschrieben. Die Gelierung und Freisetzung sind aber sehr stark.von den Umgebungsbedingungen abhängig.

In US 5,232,704 werden Formen beschrieben, die aus 2 Schichten bestehen, wovon die eine wirkstoffhaltig und die andere für das Aufschwimmen verantwortlich ist. Die Herstellung ist aufwendig und hochdosierte Arzneistoffe können nicht verarbeitet werden.

Aerogele, Schäume bzw. Luft enthaltende Mikrokapseln sind ebenfalls beschrieben, in WO 96/25950 (Hoechst AG), EP 326816 (LTS Lohmann), WO 95/05809 (Nippon Shinyaku), jedoch besteht bei diesen Mikrokapseln oft der Nachteil, daß die Stoffe nicht pharmazeutisch zugelassen sind, sehr aufwendig zu verarbeiten sind bzw. schlecht tablettierbar sind. Die Lyophilisierung von Wirk- oder Zusatzstoffen zur Erzeugung eines porösen Formkörpers ist enorm zeit- und kostenintensiv.

In der EP-A 0575930 sind feste pharmazeutische Retardformen beschrieben, die unter Verwendung eines redispergierbaren Pulvers, welches durch Sprühtrocknung in Gegenwart von Copolymeren aus N-Vinylpyrrolidon und Vinylacetat erhalten wird, hergestellt werden können.

Werden poröse Formkörper als Ausgangsmaterial verwendet und darauf Wirkstoff bzw. weitere Hilfsstoffe aufgebracht, steigt das Volumen der Darreichungsform entsprechend an.

Auch die Verwendung von Lösungsmitteln bei der Herstellung erhöht die Kosten und ist zudem nicht umweltschonend.

Der vorliegenden Erfindung lag daher die Aufgabe zugrunde eine geeignete Zusammensetzung für eine Retard-Darreichungsform zu entwickeln, die die o.g. Nachteile nicht aufweist.

Die Aufgabe wurde erfindungsgemäß gelöst durch eine orale Darreichungsform enthaltend
a) einen oder mehrere Wirkstoffe
b) eine formulierte Mischung von Polyvinylacetat und Polyvinylpyrrolidon
c) sowie gegebenenfalls weitere zur Herstellung der Darreichungsform übliche Hilfsstoffe,
wobei die Darreichungsform auf dem Magensaft schwimmt und eine verzögerte Wirkstofffreisetzung aufweist, erhältlich durch Verpressung bei einem Pressdruck unter 100 mPa..

Bevorzugt werden die Darreichungsformen für pharmazeutische Wirkstoffe eingesetzt.

Sie können aber auch für jeden anderen Wirkstoff, bei dem eine verzögerte Freisetzung erwünscht ist eingesetzt werden.

Diese Formen können durch einfache Verfahren hergestellt werden und weisen außergewöhnliche mechanische Festigkeiten auf.- Überraschenderweise können aus Polyvinylacetat und Polyvinylpyrrolidon Schwimmformen mit einer verzögerten Freisetzung hergestellt werden, obwohl von N. Rouge, E. T. Cole, E. Doelker und P. Buri, S. T. P. Pharma Sciences 7(5), 386-92 (1997) festgestellt wurde, daß mit inerten Matrixbildnern wie Ethylcellulose und Celluloseacetat durch Verpressung keine Schwimmformen hergestellt werden können. Nur quellende Matrixbildner waren dazu in der Lage. Polyvinylacetat und Polyvinylpyrrolidon bilden bei der Verpressung ebenfalls inerte Matrices aus, d. h. es findet keine Quellung und Erosion in Magen- oder Darmsaft statt.

Die außergewöhnliche gute Verpreßbarkeit dieser Kombination ermöglicht die Herstellung von mechanisch sehr stabilen oralen Darreichungsformen, insbesondere Tabletten mit einer stark retardierten Freisetzung schon bei extrem niedrigen Preßdrücken. Durch das plastische Verhalten dieser Kombination bleibt eine hohe Porosität erhalten, wodurch die Darreichungsformen auf dem Magensaft schwimmen. Obwohl normalerweise zu erwarten war, daß das große Porenvolumen zu einer schnellen Freisetzung führt, ist trotz der hohen Porosität eine starke Retardierwirkung vorhanden.

Auch nach dem Eindringen von Magensaft schwimmen die Darreichungsformen bis zu 48 h und sinken trotz der dadurch eintretenden Gewichtszunahme nicht ab.

Überraschenderweise vereint die Kombination Polyvinylacetat-Polyvinylpyrrolidon folgende Eigenschaften:
1. Floatingwirkung durch Erzeugung einer hohen Porosität in der Tablette
2. Bindemittelwirkung, wodurch ein zusätzliches Bindemittel in der Regel überflüssig wird
3. Fließmittelwirkung, da es die Fließfähigkeit von Pulvermischungen enorm verbessert
4. Retardierungswirkung.

Das Verhältnis Polyvinylacetat zu Polyvinylpyrrolidon liegt bevorzugt zwischen 6:4 bis 9:1, insbesonders bevorzugt bei 8:2.

Durch Kombination dieser verschiedenen Wirkungen werden häufig keine weiteren Hilfsstoffe in der Darreichungsform benötigt bzw. deren Menge kann sehr klein gewählt werden. Die Folge ist eine bezüglich des Volumens sehr kleine Form, die eine einfache orale Applikation ermöglicht.

Die Einarbeitung von Salzen der Kohlensäure ist nicht erforderlich ebenso die von anderen porösen Formlingen, Hohlkugeln etc. Prinzipiell können diese Stoffe natürlich eingearbeitet werden, wobei die Mengen gering sein sollten, weil bei größeren Mengen, insbesondere von Kohlensäuresalzen, das Gefüge Schaden nehmen kann. Die Menge an Kohlensäuresalzen sollte unter 10 % vorzugsweise unter 5 % liegen, die der porösen Formkörper unter 30 % vorzugsweise unter 20 %.

Die Herstellung der schwimmenden Darreichungsformen erfolgt am einfachsten durch Direkttablettierung, wozu oft nur Wirkstoff, die Kombination aus Polyvinylacetat und Polyvinylpyrrolidon und ein Schmiermittel nötig sind. Die Rezeptur ist einfach, sehr reproduzierbar und sehr robust. Natürlich können auch weitere übliche Tablettierhilfsstoffe beispielsweise Fließregulierungsmittel, Bindemittel, Sprengmittel, Farb- oder Füllstoffe dazugegeben werden. Zur Überdeckung eines schlechten Geschmacks oder Geruchs können Aromen oder Süßstoffe zugesetzt werden.

Als Schmiermittel können Stearate von Aluminium, Calcium, Magnesium und Zinn, sowie Magnesiumsilikat, Silikone und ähnliche verwendet werden.

Fließmittel können beispielsweise sein, Talk, kolloidales Siliciumdioxid, Stärke,oder fließfähige mikrokristalline Cellulose.

Bindemittel sind z.B. Stärke, Alginate, Carboxymethylcellulose oder Polyvinylpyrrolidon. Sprengmittel können sein Stärke bzw. Stärkekleister oder mikrokristalline Cellulose. Stabilisatoren.

Als Füllstoffe können z.B. anorganische Füllstoffe wie Oxide von Magnesium, Aluminium, Silicium, Titan- oder Calciumcarbonat zugesetzt werden.

Farbstoffe sind z.B. Eisenoxide, Titandioxid, Triphenylmethanfarbstoffe, Azofarbstoffe, Chinolinfarbstoffe, Indigotinfarbstoffe, Carotinoide, um die Darreichungsformen einzufärben, Opakisierungsmittel wie Titandiodid oder Talkum, um die Lichtdurchlässigkeit zu erhöhen und um Farbstoffe einzusparen.

Auch Trockengranulationsprozesse und Feuchtgranulationsprozesse können verwendet werden. Hierbei ist darauf zu achten, daß Prozesse gewählt werden, die zu keiner starken Verdichtung führen. So ist die Wirbelschichtgranulation aufgrund des geringen mechanischen Energieeintrages ideal dafür.

Die erfindungsgemäßen Darreichungsformen eignen sich zum Einsatz in oralen Darreichungsformen wie Tabletten oder Kapseln, bevorzugt zur Herstellung von Tabletten.

Üblicherweise sind zur Herstellung von mechanisch stabilen Tabletten Preßdrücke von 150 - 800 MPa erforderlich. Für die Herstellung der Schwimmformen werden nur Drücke unter 100, - vorzugsweise unter 60 MPa benötigt. Bei solch niedrigeren Drücken entstehen mit üblichen Tablettierhilfsstoffen entweder überhaupt keine Tabletten - das Preßgut kommt als Pulver aus der Matrize - oder die Tablette weist so geringe Festigkeiten auf, daß sie nicht weiterverarbeitbar ist. Tabletten müssen so stabil sein, daß sie einen Coatingprozeß bzw. die Verpackung unbeschadet überleben. Dies ist in der Regel gegeben, wenn die auf die Bruchfläche bezogene Festigkeit oberhalb von 1 N/mm² vorzugsweise 2 N/mm² und die Friabilität kleiner als 2,0 % vorzugsweise kleiner 1,0 % ist.

Bei der Entwicklung der Schwimmformen wird der Preßdruck so weit gesteigert, daß einerseits die Tablette noch schwimmt, andererseits aber auch eine möglichst hohe Bruchfestigkeit und ein niedriger Abrieb erzielt werden. Der Abrieb sollte kleiner 3%, bevorzugt kleiner 1,5 %, besonders bevorzugt kleiner 1 % liegen.

Aufwendige Verarbeitungsprozesse wie Lyophilisation, Coating von Tablettenbestandteilen oder Schritte unter Verwendung von organ. Lösungsmitteln sind nicht erforderlich.

Die so verpreßten Tabletten können auch in Form von Mikrotabletten in Kapseln eingebracht werden.

Die erfindungsgemäßen Darreichungsformen können jeden Wirkstoff für den eine verzögerte Freisetzung erwünscht ist enthalten.

Bevorzugt werden als Wirkstoffe Nahrungsergänzungs- oder Zusatzstoffe, Vitamine, Mineralstoffe oder Spurenelemente, insbesonders bevorzugt aber pharmazeutische Wirkstoffe eingesetzt.

Pharmazeutische Formulierungen der oben genannten Art können durch Verarbeiten der beanspruchten Verbindungen mit pharmazeutischen Wirkstoffen nach herkömmlichen Methoden und unter Einsatz bekannter und neuer Wirkstoffe erhalten werden. Die Wirkstoffe können dabei aus jedem Indikationsgebiet kommen.

Als Beispiele seien hier die folgenden genannt:

Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytica, Betarezeptorenblocker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel.

Die Wirkstoffreisetzung wird durch die Menge an Polyvinylacetat/Polyvinylpyrrolidon eingestellt. Höhere Mengen an der Kombination verlangsamen die Freisetzung. Die für das Aufschwimmen und eine retardierte Freisetzung notwendigen Mengenanteile liegen zwischen 10 und 99 %, vorzugsweise zwischen 20 und 70 % bezogen auf das Gesamtgewicht der Tablette. Neben der formulierten Mischung aus Polyvinylacetat und Polyvinylpyrrolidon können auch weitere retardierende Hilfsstoffe vor oder nach der Granulation eingesetzt werden.

Die Freisetzung kann beschleunigt werden, indem leicht wasserlösliche Polymere zugesetzt werden; sie kann aber auch verzögert werden, indem sehr lipophile oder in Wasser quellende Stoffe zugesetzt werden. Letztere führen zu einer Gelbildung in den Poren der inerten Matrix und behindern so die Diffusion des Arzneistoffes nach außen. Solche Gelbildner sind beispielsweise Alginate, Pektine, Galactomannane, Carrageenane, Dextran, Curdlan, Pullulan Gellan, Chitin, Gelatine, Xanthane, Hemicellulosen, Cellolosederivate wie Methylcellulose, Hydroxypropylmethylcellulose, Hydroxypropylcellulose, Hydroxyethylcellulose, Carboxymethylcellulose, Stärkederivate wie Carboxymethylstärke, abgebaute Stärke, Maltodextrine, Polyacrylsäure, Polymethacrylsäure, Acrylsäure-Methacrylsäure-Copolymere, Polyvinylalkohole, hochmolekulare Polyethylenglykole, Polyoxyethylen-Polyoxypropylen-Blockpolymerisate, hochmolekulare Polyvinylpyrrolidone sowie Derivate davon.

Zu den lipophilen Stoffen zählen beispielsweise Fettalkohole wie Stearylalkohol, Fettsäuren wie Stearinsäure, Glyceride, Fettsäureester und Fettalkoholester, lipophile Polymere wie Ethylcellulose, Celluloseacetat, Acrylatester-Methacrylatester-Coplymerisate, Methacrylsäure-Acrylsäureester-Copolymere, Celluloseacetatphthalat, Celluloseacetatsuccinat, Hydroxypropylmethylcelluloseacetatphthalat, Hydroxypropylmethylcelluloseacetatsuccinat.

Zu den wasserlöslichen Polymeren gehören beispielsweise Polyethylenglykole, Polyvinylpyrrolidon oder Vinylpyrrolidon/Vinylacetat Copolymere.

Diese Zusatzstoffe können in Mengen von 0,1 - 30 %, vorzugsweise 0,5 - 20 % eingesetzt werden.

Die Tablettenform kann in weiten Grenzen variiert werden. So sind gewölbte, biplane, runde, kantige Tabletten herstellbar wie auch oblong- oder football-shape-Formen. Die Größe wird nach oben limitiert durch die Schluckbarkeit nach unten durch maschinenbauliche Grenzen. Übliche Tablettengrößen liegen zwischen 1 und 16 mm, vorzugsweise zwischen 2 und 13 mm Durchmesser.

Daneben lassen sich auch Zweischichttabletten herstellen, bei denen eine Schicht die gesamte Dosis an Wirkstoff enthält oder zumindest sehr wirkstoffreich ist, während die andere Schicht sehr reich ist an der Kombination Polyvinylacetat-Polyvinylpyrrolidon. Diese beiden Schichten können sich auch in ihrer Porosität unterscheiden. In der Regel ist die Polyvinylacetat-Polyvinylpyrrolidon-reiche Schicht poröser und dann hauptsächlich für das Floating verantwortlich.

Eine besondere Ausprägung ist die Herstellung von Manteltabletten, bei denen der Kern sehr wirkstoffreich ist bzw. sogar die Gesamtmenge an Wirkstoff enthalten kann, während die Hülle zu einem großen Teil aus der Kombination Polyvinylacetat-Polyvinylpyrrolidon besteht. Dadurch wird eine starke Retardierung erzeugt. Diese Form ist besonders für sehr leicht wasserlösliche Wirkstoffe, die stark retardiert werden sollen, geeignet.

Die erfindungsgemäßen Tabletten können auch durch Schmelzextrusion und anschließende Kalandrierung hergestellt werden.

Die Tabletten können in üblicher Weise mit einem Filmüberzug versehen werden. Dieses Coating kann wasserlöslich sein, dann dient es lediglich der Verbesserung des optischen Erscheinungsbildes bzw. der Überdeckung eines schlechten Geruches oder Geschmacks, es kann aber auch wasserunlösliches sein und dann zur weiteren Verringerung der Wirkstofffreisetzung verwendet werden. Dies ist notwendig, wenn eine sehr lange Wirkdauer angestrebt wird. Prinzipiell sind alle pharmazeutisch zugelassenen Coatingmaterialien einsetzbar, beispielsweise Hydroxypropylmethylcellulose (Pharmacoat 603 oder 606, Fa. Shin-Etsu), Hydroxypropylcellulose, Ethylcellulose, Celluloseacetatphthalat, Ammoniomethacrylat Copolymer (USP), Methacrylsäure Copolymer Typ C (USP), Butylmethacrylat-2-dimethylaminoethylmethacrylat-methylmethacrylat-Copolymer, Polyvinylacetat, Polyvinylpyrrolidon

Die nachfolgenden Beispiele sollen die Erfindung näher erläutern, ohne sie jedoch darauf zu beschränken.

### Beispiel 1

### Coffein-Schwimmtablette

1,6 kg Coffein, 1,6 kg formulierte Mischung aus Polyvinylacetat und Polyvinylpyrrolidon im Verhältnis 8:2 (Kollidon SR) und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 10 mm und einem Gewicht von 322 mg verpreßt. Die Preßkraft betrug 2,04 kN.

Folgende Pulver- bzw. Tablettendaten wurden ermittelt :

| | |
|---|---|
| Böschungswinkel: | 23° |
| Auslaufzeit | 7,7 s |
| Bruchfestigkeit: | 54 N |
| Abrieb: | 0,94 % |

Die Tabletten schwammen sofort nach Zugabe auf künstlichem Magensaft. Das Aufschwimmen hielt über 48 h an (Tabelle 1).

**Tabelle 1 Freisetzung Coffein Schwinantabletten**

| Zeit [h] | freigesetzte Wirkstoffmenge [%] |
|---|---|
| 0 | 0,0 |
| 0,5 | 25,6 |
| 1 | 39,4 |
| 1,5 | 53,1 |
| 2 | 61,5 |
| 3 | 71,9 |
| 4 | 80,6 |
| 6 | 96,3 |
| 8 | 100,9 |
| 12 | 103,0 |
| 16 | 103,3 |

### Beispiel 2

### Diltiazem-Schwimmtablette

1,2 kg Diltiazem-HCl, 2,0 kg formulierte Mischung aus Polyvinylacetat und Polyvinylpyrrolidon im Verhältnis 8:2 (Kollidon SR), 0,03 kg Aerosil 200 und 0,03 kg Magnesiumstearat wurden durch ein 0,8-mm-Sieb gegeben, 10 min im Turbula-Mischer gemischt und auf einer Korsch-Rundläufertablettenpresse PH 106 zu biplanen Tabletten mit einem Durchmesser von 10 mm und einem Gewicht von 326 mg verpreßt. Die Preßkraft betrug 3,76 kN.

Folgende Pulver- bzw. Tablettendaten wurden ermittelt:

| | |
|---|---|
| Böschungswinkel: | 29° |
| Auslaufzeit | 15,5 s |
| Bruchfestigkeit: | 111 N |
| Abrieb: | 0,26 % |

Die Tabletten schwammen sofort nach Zugabe auf künstlichem Magensaft. Das Aufschwimmen hielt über 48 h an (Tabelle 2-3).

**Tabelle 2 Freisetzung Diltiazem-HCl Schwimmtabletten**

| | | | | |
|---|---|---|---|---|
| Zeit [h] | freigesetzte wirkstoffmenge [%] | | | |
| 0 | 0 | 0 | 0 | 0 |
| 0.5 | 15,9 | 16,3 | 15,5 | 15,4 |
| 1 | 24,5 | 24,4 | 22,1 | 22,3 |
| 1,5 | 32,2 | 29,4 | 30,4 | 30,1 |
| 2 | 37,8 | 37,6 | 36,3 | 35,7 |
| 3 | 44,8 | 44,9 | 43,1 | 42,7 |
| 4 | 54,1 | 50,9 | 48,7 | 48,6 |
| 6 | 64,0 | 60,5 | 58,9 | 56,7 |
| 8 | 70,9 | 69,3 | 66,2 | 64,7 |
| 12 | 82,8 | 83,3 | 76,8 | 74,8 |
| 16 | 92,5 | 91,6 | 85,9 | 87,6 |

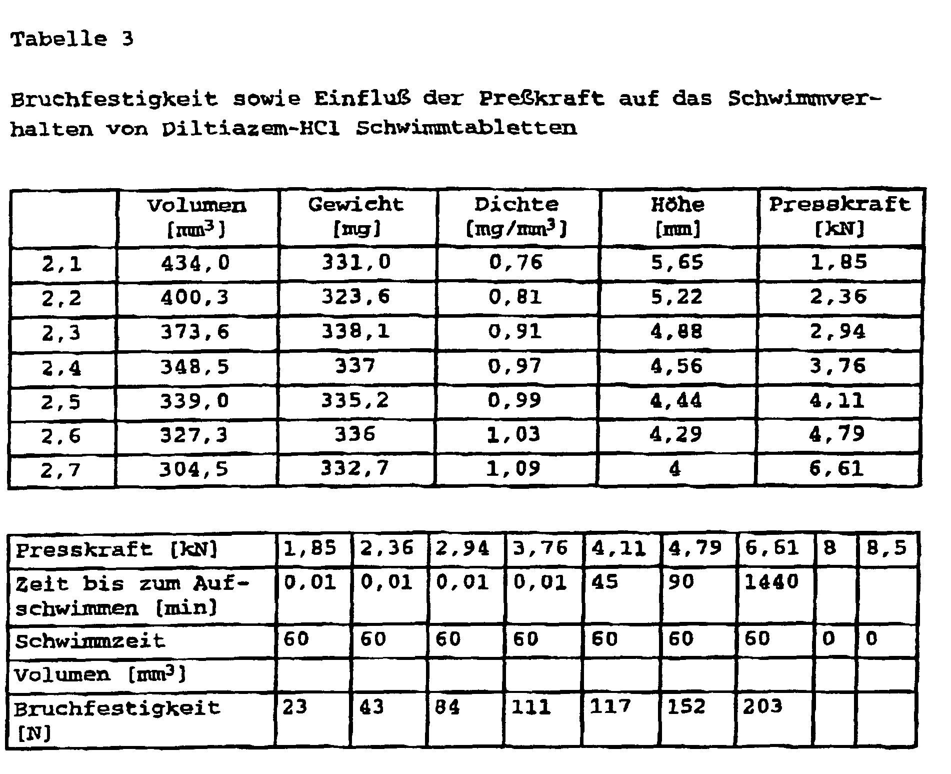

### Beispiel 3

### Tramadol-Schwimmtablette

1,0 kg Tramadol-HCl, 1,5 kg formulierte Mischung aus Polyvinylacetat und Poly-vinylpyrrolidon im Verhältnis 8:2 (Kollidon SR), 0,1 kg Xanthan, 0,03 kg Aerosil 200 und 0,03 kg Magnesiumstearat wurden durch ein 0,8-mm-Sieb gegeben, 10 min im Turbula-Mischer gemischt und auf einer Korsch-Rundläufertablettenpresse PH 106 zu biplanen Tabletten mit einem Durchmesser von 10 mm und einem Gewicht von 276 mg verpreßt. Die Preßkraft betrug 3,76 kN.

Folgende Pulver- bzw. Tablettendaten wurden ermittelt:

| | |
|---|---|
| Böschungswinkel: | 24,6° |
| Auslaufzeit | 9,2 s |
| Bruchfestigkeit: | 62 N |
| Abrieb: | 0,24 % |

Die Tabletten schwammen sofort nach Zugabe auf künstlichem Magensaft. Das Aufschwimmen hielt über 48 h an (Tabelle 4).

**Tabelle 4 Freisetzung Tramadol**

| Zeit [h] | freigesetzte Wirkstoffmenge [%] |
|---|---|
| 0 | 0,0 |
| 0,5 | 25,6 |
| 1 | 39,4 |
| 1,5 | 53,1 |
| 2 | 61,5 |
| 3 | 71,9 |
| 4 | 80,6 |
| 6 | 96,3 |
| B | 100,9 |
| 12 | 103,0 |
| 16 | 103,3 |

### Beispiel 4

### Propranolol-HCl-Schwimmtablette

1,6 kg Propranolol-HCl und 1,6 kg formulierte Mischung aus Polyvinylacetat und Polyvinylpyrrolidon im Verhältnis 8:2 (Kollidon SR) wurden durch ein 0,8-mm-Sieb gegeben und in einem Wirbelschichtgranulator durch Besprühen mit 0,7 kg demineralisiertem Wasser bei einer Zulufttemperatur von 50 °C granuliert. Dem getrockneten Granulat wurden im Turbula-Mischer 0,03 kg Magnesiumstearat bei einer Mischzeit von 10 min zugemischt. Diese Mischung wurde auf einer Korsch-Rundläufertablettenpresse PH 106 zu biplanen Tabletten mit einem Durchmesser von 10 mm und einem Gewicht von 320 mg verpreßt. Die Preßkraft betrug 3,14 kN.

Folgende Pulver- bzw. Tablettendaten wurden ermittelt:

| | |
|---|---|
| Böschungswinkel: | 29,5° |
| Auslaufzeit | 9,8 s |
| Bruchfestigkeit: | 59 N |
| Abrieb: | 0,49 % |

Die Tabletten schwammen sofort nach Zugabe auf künstlichem Magensaft. Das Aufschwimmen hielt über 48 h an (Tabelle 5),

**Tabelle 5 Freisetzung Propanolol Schwimmtabletten**

| Zeit [h] | freigesetzte Wirkstoffmenge [%] |
|---|---|
| 0 | 0,0 |
| 0,5 | 21,5 |
| 1 | 31,2 |
| 1.5 | 37,5 |
| 2 | 42,8 |
| 3 | 49,5 |
| 4 | 57,8 |
| 6 | 73,0 |
| 8 | 83,5 |
| 12 | 92,7 |
| 16 | 97,5 |

### Beispiel 5

### Gecoatete Diltiazem-Schwimmtablette

1,2 kg Diltiazem-HCl, 2,0 kg formulierte Mischung aus Polyvinylacetat und Polyvinylpyrrolidon im Verhältnis 8:2 (Kollidon SR) und 0,03 kg Magnesiumstearat wurden durch ein 0,8-mm-Sieb gegeben, 10 min im Turbula-Mischer gemischt und auf einer Korsch-Rundläufertablettenpresse PH 106 zu gewölbten Tabletten mit einem Durchmesser von 9 mm und einem Gewicht von 323 mg verpreßt. Die Preßkraft betrug 3,54 kN.

Diese Tablettenkerne wurden in einem Horizontaltrommelcoater (Accela Cota, Fa. Manesty) mit einer Lösung von 160 g Hydroxypropylmethylcellulose (Pharmacoat 606) in 2,0 kg demineralisiertem Wasser bei einer Zulufttemperatur von 50 °C gecoatet.

Folgende Tablettendaten wurden ermittelt:

| | |
|---|---|
| Bruchfestigkeit: | 124 N |
| Abrieb: | 0,11 % |

Die Tabletten schwammen sofort nach Zugabe auf künstlichem Magensaft. Das Aufschwimmen hielt über 48 h an.

### Beispiel 6

### Theophyllin-Mikroschwimmtabletten

1,6 kg Theophyllin, 1,6 kg formulierte Mischung aus Polyvinylacetat und Polyvinyl-pyrrolidon im Verhältnis 8:2 (Kollidon SR) und 0,02 kg Magnesiumstearat wurden durch ein 0,8-mm-Sieb gegeben, 10 min im Turbula-Mischer gemischt und auf einer Korsch-Excentertablettenpresse EKO zu gewölbten Tabletten mit einem Durchmesser von 2 mm und einem Gewicht von 6 mg verpreßt. Die Preßkraft betrug unter Verwendung eines 5fach-Werkzeuges 0,4 kN. 50 Mikrotabletten wurden in eine Kapsel der Größe 0 elongated gefüllt.

Die Tabletten wiesen einen Abrieb von 0,5 % auf und sie schwammen nach Auflösen der Kapsel sofort auf künstlichem Magensaft. Das Aufschwimmen hielt über 24 h an.

### Beispiel 7

### Coffein-Schwimmtablette

1,6 kg Coffein, 1,6 kg formulierte Mischung aus Polyvinylacetat und Polyvinylpyrrolidon im Verhältnis 7:3, 0,2 kg Hydroxypropylmethylcellulose (Methocel K 100) und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 10 mm und einem Gewicht von 322 mg verpreßt. Die Preßkraft betrug 2,82 kN.

Folgende Pulver- bzw. Tablettendaten wurden ermittelt:

| | |
|---|---|
| Böschungswinkel: | 28° |
| Auslaufzeit | 13,6 s |
| Bruchfestigkeit: | 56 N |
| Abrieb: | 0,82 % |

Die Tabletten schwammen sofort nach Zugabe auf künstlichem Magensaft. Das Aufschwimmen hielt über 48 h an.

### Beispiel 8

### Diltiazem-Schwimmtablette

1,6 kg Diltiazem-HCl, 1,6 kg formulierte Mischung aus Polyvinylacetat und Polyvinylpyrrolidon im Verhältnis 8:2, 0,03 kg Natriumhydrogencarbonat fein gepulvert und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 10 mm und einem Gewicht von 325 mg verpreßt. Die Preßkraft betrug 4,15 kN.

Folgende Pulver- bzw. Tablettendaten wurden ermittelt:

| | |
|---|---|
| Böschungswinkel: | 30° |
| Auslaufzeit | 15,9 s |
| Bruchfestigkeit: | 139 N |
| Abrieb: | 0,09 % |

Die Tabletten schwammen sofort nach Zugabe auf künstlichem Magensaft. Das Aufschwimmen hielt über 48 h an.

### Beispiel 9

### Biperiden -Schwimmtablette

0,04 kg Biperiden-HCl, 1,6 kg formulierte Mischung aus Polyvinylacetat und Poly-vinylpyrrolidon im Verhältnis 8:2, 0,34 kg mikrokristalline Cellulose und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 8 mm und einem Gewicht von 200 mg verpreßt. Die Preßkraft betrug 1,9 kN.

Folgende Pulver- bzw. Tablettendaten wurden ermittelt:

| | |
|---|---|
| Böschungswinkel: | 24° |
| Auslaufzeit | 7,3 s |
| Bruchfestigkeit: | 78 N |
| Abrieb: | 0,04 % |

Die Tabletten schwammen sofort nach Zugabe auf künstlichem Magensaft. Das Aufschwimmen hielt über 48 h an.

### Vergleichsbeispiel

### Coffeintablette mit Hydroxypropylmethylcellulose

1,6 kg Coffein, 1,6 kg Hydroxypropylmethylcellulose (Methocel K 100) und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 10 mm und einem Gewicht von 322 mg verpreßt. Die Preßkraft betrug 2,06 kN.

Folgende Pulver- bzw. Tablettendaten wurden ermittelt:

| | |
|---|---|
| Böschungswinkel: | 42° |
| Auslaufzeit | stockts |
| Bruchfestigkeit: | 7N |
| Abrieb: | 100% (Bruch) |

Die Tabletten schwammen sofort nach Zugabe auf künstlichem Magensaft aber die mechanischen Eigenschaften sind völlig unzureichend.

Bei einer Preßkraft von 6,48 kN wurde eine Bruchfestigkeit von 49 N gemessen, aber die Tabletten schwimmen nicht mehr auf künstlichem Magensaft.

### Vergleichsbeispiel

### Coffeintablette mit Acrylatestercopolymer

1,6 kg Coffein, 1,6 kg Eudragit RS und 0,02 kg Magnesiumstearat wurden durch ein 0,8-mm-Sieb gegeben, 10 min im Turbula-Mischer gemischt und auf einer Korsch-Excentertablettenpresse EKO zu biplanen Tabletten mit einem Durchmesser von 10 mm und einem Gewicht von 322 mg verpreßt. Die Preßkraft betrug 3,03 kN.

Folgende Pulver- bzw. Tablettendaten wurden ermittelt:

| | |
|---|---|
| Böschungswinkel: | 41° |
| Auslaufzeit | 10,4s |
| Bruchfestigkeit: | 5N |
| Abrieb: | 100% (Bruch) |

Die Tabletten sinken sofort nach Zugabe auf künstlichen Magensaft zu Boden und die mechanischen Tabletteneigenschaften sind völlig unzureichend.

Selbst bei einer Preßkraft von 6,04 kN wurde nur eine Bruchfestigkeit von 14 N gemessen.

## Patentansprüche

1. Orale Darreichungsformen enthaltend
a) einen oder mehrere Wirkstoffe
b) eine formulierte Mischung von Polyvinylacetat und Polyvinylpyrrolidon
c) gegebenenfalls weitere zur Herstellung der Darreichungsform übliche Hilfsstoffe,
wobei die Darreichungsformen auf dem Magensaft schwimmen und eine verzögerte Wirkstofffreisetzung aufweisen, erhältlich durch Verpressung bei einem Pressdruck unter 100 MPa.

2. Darreichungsform gemäß Anspruch 1, **dadurch gekennzeichnet, daß** das Verhältnis Polyvinylacetat zu Polyvinylpyrrolidon 6:4 bis 9:1 beträgt.

3. Darreichungsform gemäß einem der Ansprüche 1 oder 2, **dadurch gekennzeichnet, daß** es sich bei der oralen Darreichungsform um eine Tablette, oder eine überzogene Tablette handelt.

4. Darreichungsform gemäß einem der Ansprüche 1-3, **dadurch gekennzeichnet, daß** es sich bei der Darreichungsform um eine Tablette handelt.

5. Darreichungsform gemäß einem der Ansprüche 1-4, **dadurch gekennzeichnet, daß** der Anteil der formulierten Mischung aus Polyvinylacetat und Polyvinylpyrrolidon zwischen 10 und 99 %, liegt, bezogen auf das Tablettengesamtgewicht, vorzugsweise zwischen 20 und 70 %.

6. Darreichungsform gemäß einem der Ansprüche 1-5, **dadurch gekennzeichnet, daß** der Abrieb kleiner 3 % beträgt.

7. Darreichungsform gemäß einem der Ansprüche 1-6, **dadurch gekennzeichnet, daß** ein wasserlösliches oder wasserunlösliches, retardierendes Coating auf die orale Darreichungsform aufgebracht wird.

8. Darreichungsform gemäß einem der Ansprüche 1-7, **dadurch gekennzeichnet, daß** zur Einstellung der Freisetzung leicht wasserlösliche, wasserlösliche, starkquellende oder lipophile Stoffe zugesetzt werden.

9. Darreichungsform gemäß Anspruch 8, **dadurch gekennzeichnet, daß** die wasserlöslichen stark quellende Stoffe ausgewählt sind aus der Gruppe der Alginate, Pektine, Galactomannane, Carrageenane, Dextran, Curdlan, Pullulan Gellan, Chitin, Gelatine, Xanthane, Hemicellulosen, Cellolosederivate wie Methylcellulose, Hydroxypropylmethylcellulose, Hydroxypropylcellulose, Hydroxyethylcellulose, Carboxymethylcellulose, Stärkederivate wie Carboxymethylstärke,abgebaute Stärke, Maltodextrine, Polyacrylsäure, Polymethacrylsäure Acrylsäure-Methacrylsäure-Copolymere, Polyvinylalkohole, hochmolekulare Polyethylenglykole, Polyoxyethylen-Polyoxypropylen-Blockpolymerisate, hochmolekulare Polyvinylpyrrolidone sowie Derivate davon und als lipophile Stoffe Fettalkohole wie Stearylalkohol, Fettsäuren wie Stearinsäure, Glyceride, Fettsäureester und Fettalkoholester, lipophile Polymere wie Ethylcellulose, Celluloseacetat, Acrylatester-Methacrylatester-Copolymerisate, Methacrylsäure-Acrylsäureester-Copolymere, Celluloseacetatphthalat, Celluloseacetatsuccinat, Hydroxypropylmethylcelluloseacetatphthalat, Hydroxypropylmethylcelluloseacetatauccinat.

10. Darreichungsform gemäß Anspruch 8, **dadurch gekennzeichnet, daß** die wasserlöslichen Stoffe ausgewählt sind aus der Gruppe der Polyethylenglykole, Polyvinylpyrrolidon oder Vinylpyrrolidon/Vinylacetat Copolymere.

11. Darreichungsform gemäß Anspruch 8, **dadurch gekennzeichnet, daß** die lipophilen Stoffe ausgewählt sind aus der Gruppe der Fettalkohole wie Stearylalkohol, Fettsäuren wie Stearinsäure, Glyceride, Fettsäureester und Fettalkoholester, lipophile Polymere wie Ethylcellulose, Celluloseacetat, Acrylatester-Methacrylatester-Coplymerisate. Methacrylsäure-Acrylsäureester-Copolymere, Celluloseacetatphthalat, Celluloseacetatsuccinat, Hydroxypropylmethylcelluloseacetatphthalat, Hydroxypropylmethylcelluloseacetatsuccinat.

12. Darreichungsform gemäß einem der Ansprüche 1-11, **dadurch gekennzeichnet, daß** sie als weitere Hilfsstoffe c), Schmiermittel, Bindemittel, Sprengmittel, Fließregulierungsmittel, Stabilisatoren, Farbstoffe oder Füllstoffe enthält.

13. Darreichungsform gemäß einem der Ansprüche 1-12, **dadurch gekennzeichnet, daß** sie als Wirkstoffe a) Nahrungsergänzungs- oder Zusatzstoffe, Vitamine, Mineralstoffe oder Spurenelemente oder pharmazeutische Wirkstoffe enthält.

14. Darreichungsform gemäß einem der Ansprüche 1-13, **dadurch gekennzeichnet, daß** sie als Wirkstoffe a) pharmazeutische wirkstoffe enthält.

15. Darreichungsform gemäß einem der Ansprüche 1-14, **dadurch gekennzeichnet, daß** der pharmazeutische Wirkstoff ausgewählt ist aus der Gruppe der Benzodiazepine, Antihypertensiva, Vitamine, Cytostatika, Anästhetika, Neuroleptika, Antidepressiva, Antibiotika, Antimykotika, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Trausportproteine, Enzyminhibitoren, Emetika, Durchblutungsfördernde Mittel, Diuretika, Diagnostika, Corticoide, Cholinergika, Gallenwegstherapeutika. Antiasthmatika, Broncholytica, Betarezeptorenblokker, Calciumantagonisten, ACE-Hemmer, Arteriosklerosemittel, Antiphlogistika, Antikoagulatia, Antihypotonika, Antihypoglykämika, Antihypertonika, Antifibrinolytika, Antiepileptika,Antiemetika, Antidota, Antidiabetika, Antiarrhythmika, Antianämika, Antiallergika, Anthelmintika, Analgetika, Analeptika. Aldosteronantagonisten, Abmagerungsmittel.

16. Darreichungsform gemäß einem der Ansprüche 1-15, **dadurch gekennzeichnet, daß** zwei oder mehrere Schichten vorhanden sind, die sich in dem Gehalt an Wirkstoff und formulierter Mischung aus Polyvinyacetat und Polyvinylpyrrolidon unterscheiden.

17. Darreichungsform gemäß einem der Ansprüche 1-16, **dadurch gekennzeichnet, daß** es sich um eine Manteltablette mit sehr wirkstoffreichem Kern handelt.

18. Verfahren zur Herstellung von oralen Darreichungsformen gemäß einem der Ansprüche 1-17, **dadurch gekennzeichnet, daß** zur Verpressung ein Preßdruck unter 100 MPa verwendet wird.

19. Verfahren zur Herstellung von oralen Darreichungsformen gemäß Anspruche 18 **dadurch gekennzeichnet, daß** die Ausgangsmischung direkttablettiert oder die Ausgangsmischung bzw. ein Teil derselben vor der Tablettierung feuchtgranuliert oder trokkenkompaktiert wird.

20. Arzneimittel zur verzögerten Wirkstofffreisetzung, **dadurch gekennzeichnet, daß** es sich um eine orale Darreichungsform gemäß einem der Ansprüche 1-17 handelt.

21. Verwendung der oralen Darreichungsform gemäß einem der Patentansprüche 1-17 zur Herstellung von Arzneimitteln mit verzögerter Wirkstofffreisetzung zur Behandlung von Krankheiten.

## Claims

1. An oral dosage form comprising
a) one or more active ingredients
b) a formulated mixture of polyvinyl acetate and polyvinylpyrrolidone
c) where appropriate other excipients customary for producing the dosage form,
where the dosage form floats on gastric fluid and displays delayed release of active ingredient, obtainable by compression using a pressure of less than 100MPa.

2. The dosage form according to claim 1, wherein the ratio of polyvinyl acetate to polyvinylpyrrolidone is from 6:4 to 9:1.

3. The dosage form according to either of claims 1 or 2, wherein the oral dosage form is a tablet or a coated tablet.

4. The dosage form according to any of claims 1-3, wherein the dosage form is a tablet.

5. The dosage form according to any of claims 1-4, wherein the proportion of the formulated mixture of polyvinyl acetate and polyvinylpyrrolidone is between 10 and 99%, based on the total weight of the tablet, preferably between 20 and 70%.

6. The dosage form according to any of claims 1-5, wherein the friability is less than 3%.

7. The dosage form according to any of claims 1-6, wherein a water-soluble or water-insoluble, release-slowing coating is applied to the oral dosage form.

8. The dosage form according to any of claims 1-7, wherein the release is adjusted by adding substances which are freely soluble in water, soluble in water, highly swelling or lipophilic.

9. The dosage form according to claim 8, wherein the highly swelling substances which are soluble in water are selected from the group of alginates, pectins, galactomannans, carrageenans, dextran, curdlan, pullulan, gellan, chitin, gelatin, xanthans, hemicelluloses, cellulose derivatives such as methylcellulose, hydroxypropylmethylcellulose, hydroxypropylcellulose, hydroxyethylcellulose, carboxymethylcellulose, starch derivatives such as carboxymethyl starch, degraded starch, maltodextrins, polyacrylic acid, polymethacrylic acid, acrylic acid/methacrylic acid copolymers, polyvinyl alcohols, high molecular weight polyethylene glycols, polyoxyethylene/polyoxypropylene block copolymers, high molecular weight polyvinylpyrrolidones, and derivatives thereof, and as lipophilic substances fatty alcohols such as stearyl alcohol, fatty acids such as stearic acid, glycerides, fatty acid esters and fatty alcohol esters, lipophilic polymers such as ethylcellulose, cellulose acetate, acrylate ester/methacrylate ester copolymers, methacrylic acid/acrylic ester copolymers, cellulose acetate phthalate, cellulose acetate succinate, hydroxypropylmethylcellulose acetate phthalate, hydroxypropylmethylcellulose acetate succinate.

10. The dosage form according to claim 8, wherein the substances which are soluble in water are selected from the group of polyethylene glycols, polyvinylpyrrolidone or vinylpyrrolidone/vinyl acetate copolymers.

11. The dosage form according to claim 8, wherein the lipophilic substances are selected from the group of fatty alcohols such as stearyl alcohol, fatty acids such as stearic acid, glycerides, fatty acid esters and fatty alcohol esters, lipophilic polymers such as ethylcellulose, cellulose acetate, acrylate ester/methacrylate ester copolymers, methacrylic acid/acrylic ester copolymers, cellulose acetate phthalate, cellulose acetate succinate, hydroxypropylmethylcellulose acetate phthalate, hydroxypropylmethylcellulose acetate succinate.

12. The dosage form according to any of claims 1-11, which comprises as other excipients c) lubricants, binders, disintegrants, flow regulators, stabilizers, colorants or bulking agents.

13. The dosage form according to any of claims 1-12, which comprises as active ingredients a) food supplements or additives, vitamins, minerals or trace elements or active pharmaceutical ingredients.

14. The dosage form according to any of claims 1-13, which comprises as active ingredients a) active pharmaceutical ingredients.

15. The dosage form according to any of claims 1-14, wherein the active pharmaceutical ingredient is selected from the group of benzodiazepines, antihypertensives, vitamins, cytostatics, anesthetics, neuroleptics, antidepressants, antibiotics, antimycotics, fungicides, chemotherapeutics, urologicals, platelet aggregation inhibitors, sulfonamides, spasmolytics, hormones, immunoglobulins, sera, thyroid therapeutics, psychopharmaceuticals, antiparkinson agents and other antihyperkinetics, ophthalmologicals, neuropathy products, calcium metabolism regulators, muscle relaxants, anesthetics, lipid-lowering agents, liver therapeutics, coronary agents, cardiac agents, immunotherapeutics, regulatory peptides and their inhibitors, hypnotics, sedatives, gynecologicals, antigout agents, fibrinolytics, enzyme products and transport proteins, enzyme inhibitors, emetics, perfusion promoters, diuretics, diagnostics, corticoids, cholinergics, biliary therapeutics, antiasthmatics, bronchospasmolytics, beta-receptor blockers, calcium channel blockers, ACE inhibitors, arteriosclerosis remedies, antiinflammatory agents, anticoagulates, antihypotensives, antihypoglycemics, antihypertensives, antifibrinolytics, antiepileptics, antiemetics, antidotes, antidiabetics, antiarrhythmics, antianemics, antiallergics, anthelmintics, analgesics, analeptics, aldosterone antagonists, weight-reducing agents.

16. The dosage form according to any of claims 1-15, wherein two or more layers are present and differ in the content of active ingredient and formulated mixture of polyvinyl acetate and polyvinylpyrrolidone.

17. The dosage form according to any of claims 1-16, which is a press-coated tablet with a core with a very high active ingredient content.

18. A process for producing oral dosage forms according to any of claims 1-17, which comprises using a pressure of less than 100 MPa for the compression.

19. A process for producing oral dosage forms according to claim 18, wherein the initial mixture is tableted directly or the initial mixture or a part thereof is wet-granulated or dry-compacted before the tableting.

20. A drug product for delayed release of active ingredient, which is an oral dosage form according to any of claims 1-17.

21. The use of the oral dosage form according to any of claims 1-17 for producing drug products with delayed release of active ingredient for treating diseases.

## Revendications

1. Formes de préparation orales, contenant
a) une ou plusieurs substances actives,
b) un mélange formulé d'acétate de polyvinyle et de polyvinylpyrrolidone,
c) éventuellement d'autres adjuvants usuels pour la production de la forme de préparation,
les formes de préparation flottant sur le suc gastrique et présentant une libération retardée de substance active, que l'on peut obtenir par pressage à une pression inférieure à 100 MPa.

2. Forme de préparation suivant la revendication 1, **caractérisée en ce que** le rapport entre acétate de polyvinyle et polyvinylpyrrolidone est de 6/4 à 9/1.

3. Forme de préparation suivant l'une des revendications 1 ou 2, **caractérisée en ce que**, pour ce qui concerne la forme de préparation orale, il s'agit d'un comprimé ou d'un comprimé revêtu.

4. Forme de préparation suivant l'une des revendications 1 à 3, **caractérisée en ce que**, pour ce qui concerne la forme de préparation, il s'agit d'un comprimé.

5. Forme de préparation suivant l'une des revendications 1 à 4, **caractérisée en ce que** la fraction du mélange formulé d'acétate de polyvinyle et de polyvinylpyrrolidone est située entre 10 et 99 %, par rapport au poids total de comprimé, de préférence entre 20 et 70 %.

6. Forme de préparation suivant l'une des revendications 1 à 5, **caractérisée en ce que** l'abrasion est inférieure à 3 %.

7. Forme de préparation suivant l'une des revendications 1 à 6, **caractérisée en ce qu'**un revêtement retardateur soluble dans l'eau ou insoluble dans l'eau est appliqué sur la forme de préparation orale.

8. Forme de préparation suivant l'une des revendications 1 à 7, **caractérisée en ce que**, pour ajuster la libération, des substances aisément solubles dans l'eau, solubles dans l'eau, fortement gonflantes ou lipophiles sont ajoutées.

9. Forme de préparation suivant la revendication 8, **caractérisée en ce que** les substances fortement gonflantes, solubles dans l'eau, sont choisies parmi le groupe des alginates, des pectines, des galactomannanes, des carragheenanes, du dextrane, du Curdlan, du Pullulan Gellan, de la chitine, des gélatines, des xanthanes, des hémicelluloses, des dérivés de cellulose tels que de la méthylcellulose, de l'hydroxypropylméthylcellulose, de l'hydroxypropylcellulose, de l'hydroxyéthylcellulose, de la carboxyméthylcellulose, des dérivés d'amidon tels que de l'amidon carboxyméthylique, de l'amidon dégradé, des maltodextrines, de l'acide polyacrylique, de l'acide polyméthacrylique, des copolymères d'acide acrylique-acide méthacrylique, des alcools polyvinyliques, des polyéthylèneglycols de haut poids moléculaire, des copolymères blocs de polyoxyéthylènepolyoxypropylène, des polyvinylpyrrolidones de haut poids moléculaire ainsi que de leurs dérivés, et, comme substances lipophiles, des alcools gras, tels que de l'alcool stéarylique, des acides gras, tels que de l'acide stéarique, des glycérides, des esters d'acides gras et des esters d'alcools gras, des polymères lipophiles tels que de l'éthylcellulose, de l'acétate de cellulose, des copolymères d'acrylate-méthacrylate, des copolymères d'esters d'acide méthacrylique-acide acrylique, de l'acétophtalate de cellulose, de l'acétosuccinate de cellulose, de l'acétophtalate d'hydroxypropylméthylcellulose, de l'acétosuccinate d'hydroxypropylméthylcellulose.

10. Forme de préparation suivant la revendication 8, **caractérisée en ce que** les substances solubles dans l'eau sont choisies parmi le groupe des polyéthylèneglycols, de la polyvinylpyrrolidone ou des copolymères de vinylpyrrolidone/acétate de vinyle.

11. Forme de préparation suivant la revendication 8, **caractérisée en ce que** les substances lipophiles sont choisies parmi le groupe des alcools gras, tels que de l'alcool stéarylique, des acides gras, tels que de l'acide stéarique, des glycérides, des esters d'acides gras et des esters d'alcools gras, des polymères lipophiles tels que de l'éthylcellulose, de l'acétate de cellulose, des copolymères d'acrylate-méthacrylate, des copolymères d'esters d'acide méthacrylique-acide acrylique, de l'acétophtalate de cellulose, de l'acétosuccinate de cellulose, de l'acétophtalate d'hydroxypropylméthylcellulose, de l'acétosuccinate d'hydroxypropylméthylcellulose.

12. Forme de préparation suivant l'une des revendications 1 à 11, **caractérisée en ce qu'**elle contient, comme autres adjuvants c), des lubrifiants, des liants, des agents de libération, des agents de régulation de l'écoulement, des stabilisants, des colorants et des substances de remplissage.

13. Forme de préparation suivant l'une des revendications 1 à 12, **caractérisée en ce qu'**elle contient, comme substances actives a), des suppléments ou compléments alimentaires, des vitamines, des substances minérales ou des oligoéléments ou des substances actives pharmaceutiques.

14. Forme de préparation suivant l'une des revendications 1 à 13, **caractérisée en ce qu'**elle contient, comme substances actives a), des substances actives pharmaceutiques.

15. Forme de préparation suivant l'une des revendications 1 à 14, **caractérisée en ce que** la substance active pharmaceutique est choisie parmi le groupe des benzodiazépines, des antihypertenseurs, des vitamines, des cytostatiques, des anesthésiques, des neuroleptiques, des antidépresseurs, des antibiotiques, des antimycotiques, des fongicides, des substances chimiothérapeutiques, des urologiques, des inhibiteurs d'agrégation des thrombocytes, des sulfonamides, des spasmolytiques, des hormones, des immunoglobulines, des sérums, des substances thérapeutiques pour la glande thyroïde, des substances psychopharmaceutiques, des agents pour la maladie de Parkinson et d'autres antihypercinétiques, des substances ophtalmiques, des préparations de neuropathie, des régulateurs du métabolisme du calcium, des relaxants musculaires, des narcotiques, des agents diminuant les lipides, des substances thérapeutiques pour le foie, des produits coronariens, des agents cardiaques, des substances immunothérapeutiques, des peptides régulateurs et leurs inhibiteurs, des hypnotiques, des sédatifs, des matières gynécologiques, des produits contre l'arthrite, des fibrinolytiques, des préparations enzymatiques et des protéines de transport, des inhibiteurs enzymatiques, des émétiques, des produits favorisant l'irrigation sanguine, des diurétiques, des produits de diagnostic, des corticoïdes, des cholinergiques, des substances thérapeutiques pour les voies biliaires, des antiasthmatiques, des broncholytiques, des agents bêta-bloquants, des antagonistes du calcium, des inhibiteurs ACE, des produits pour l'artériosclérose, des antiphlogistiques, des anticoagulants, des antihypotoniques, des antihypoglycémiques, des antihypertoniques, des antifibrinolytiques, des antiépileptiques, des antiémétiques, des antidotes, des antidiabétiques, des antiarythmiques, des antianémiques, des antiallergiques, des anthelminthiques, des analgésiques, des analeptiques, des antagonistes de l'aldostérone, des produits d'amaigrissement.

16. Forme de préparation suivant l'une des revendications 1 à 15, **caractérisée en ce que** deux ou plusieurs couches sont présentes qui se différencient dans la teneur en substance active et en mélange formulé d'acétate de polyvinyle et de polyvinylpyrrolidone.

17. Forme de préparation suivant l'une des revendications 1 à 16, **caractérisée en ce que**, pour ce qui concerne un comprimé à enveloppe, il s'agit d'un noyau très riche en substance active.

18. Procédé de production de formes de préparation orales suivant l'une des revendications 1 à 17, **caractérisé en ce que**, pour le pressage, on utilise une pression inférieure à 100 MPa.

19. Procédé de production de formes de préparation orales suivant la revendication 18, **caractérisé en ce que** le mélange de départ est directement comprimé ou le mélange de départ ou respectivement une partie de celui-ci est granulé à l'état humide ou compacté à sec avant la mise en comprimé.

20. Médicament destiné à une libération retardée de substance active, **caractérisé en ce qu'**il s'agit d'une forme de préparation orale suivant l'une des revendications 1 à 17.

21. Utilisation de la forme de préparation orale suivant l'une des revendications 1 à 17, pour la préparation de médicaments présentant une libération retardée de substance active en vue du traitement de maladies.
